# EUROPEAN PATENT SPECIFICATION

(11) **EP 3 703 697 B1**
(45) Date of publication and mention of the grant of the patent: **19.01.2022**
(21) Application number: 18803786.5
(22) Date of filing: 30.10.2018
(51) Int. Cl.: A61K 31/55, A61P 13/12, A61P 7/00

(54) **USE OF FENOLDOPAM FOR THE TREATMENT OF HEMOLYTIC UREMIC SYNDROME**
VERWENDUNG VON FENOLDOPAM ZUR BEHANDLUNG DES HÄMOLYTISCH-URÄMISCHEN SYNDROMS
UTILISATION DU FENOLDOPAM POUR LE TRAITEMENT DU SYNDROME HÉMOLYTIQUE ET URÉMIQUE

(30) Priority: 31.10.2017 IT 201700123899
(43) Date of publication of application: 09.09.2020
(73) Proprietor: Fondazione IRCCS Ca' Granda - Ospedale Maggiore Policlinico, 20122 Milano (IT)
(72) Inventor: ARDISSINO, Gianluigi, 13043 Cigliano (VC) (IT)
(74) Representative: Calogero, Ida
(86) International application number: PCT/IB2018/058466
(87) International publication number: WO 2019/087049

(56) References cited:
- BUNCHMAN TIMOTHY E: "Treatment of acute kidney injury in children: from conservative management to renal replacement therapy.", NATURE CLINICAL PRACTICE. NEPHROLOGY SEP 2008, vol. 4, no. 9, September 2008 (2008-09), pages 510-514, XP002782103, ISSN: 1745-8331
- MENNE J ET AL: "Treatment of typical hemolytic-uremic syndrome ; Knowledge gained from analyses of the 2011outbreak ; Therapie des typischen hämolytisch-urämischen Syndroms ; Erkenntnisse aus dem--Ausbruch 20", DER INTERNIST ; ORGAN DES BERUFSVERBANDES DEUTSCHER INTERNISTEN ORGAN DER DEUTSCHEN GESELLSCHAFT FÜR INNERE MEDIZIN, SPRINGER, BERLIN, DE, vol. 53, no. 12, 22 November 2012 (2012-11-22), pages 1420-1430, XP035146482, ISSN: 1432-1289, DOI: 10.1007/S00108-012-3107-5
- ARDISSINO GIANLUIGI ET AL: "Complement functional tests for monitoring eculizumab treatment in patients with atypical hemolytic uremic syndrome: an update", PEDIATRIC NEPHROLOGY, SPRINGER VERLAG, BERLIN, DE, vol. 33, no. 3, 18 October 2017 (2017-10-18), pages 457-461, XP036417113, ISSN: 0931-041X, DOI: 10.1007/S00467-017-3813-2 [retrieved on 2017-10-18]
- JOHANNA SCHEIRING ET AL: "Treatment and outcome of Shiga-toxin-associated hemolytic uremic syndrome (HUS)", PEDIATRIC NEPHROLOGY ; JOURNAL OF THE INTERNATIONAL PEDIATRIC NEPHROLOGY ASSOCIATION, SPRINGER, BERLIN, DE, vol. 23, no. 10, 13 August 2008 (2008-08-13), pages 1749-1760, XP019632034, ISSN: 1432-198X
- WIJNSMA KIOA L ET AL: "Unusual severe case of hemolytic uremic syndrome due to Shiga toxin 2d-producingE. coliO80:H2", PEDIATRIC NEPHROLOGY, SPRINGER VERLAG, BERLIN, DE, vol. 32, no. 7, 25 March 2017 (2017-03-25) , pages 1263-1268, XP036240886, ISSN: 0931-041X, DOI: 10.1007/S00467-017-3642-3 [retrieved on 2017-03-25]

## Description

The present invention relates to the use of fenoldopam, and/or its salts for the treatment of hemolytic uremic syndrome (HUS), preferably in the form due to infection from Shiga-like toxin-producing Escherichia Coli and in the atypical form.

Hemolytic uremic syndrome (HUS) is a severe clinical condition characterized by platelet consumption, hemolysis and multi-organ hypoxic/ischemic damage (particularly kidney and brain). Its parainfectious variant (the most common form) results from a gastrointestinal infection (hemorrhagic enteritis) from Shiga-like toxin-producing Escherichia Coli (STEC) (1). Its incidence varies notably due to geographic and climatic elements and in Western countries it is the main cause of acute kidney failure in the paediatric age with an average incidence (referring to Northern Italy) of 5.5 cases per million in the paediatric population (2). Although the disease is more common in children, even adults can be susceptible to it as highlighted by a recent epidemic in Germany with more than 800 affected of which 88% were adults (3).

To date the disease is encumbered by a mortality rate of 3-5% and by a risk of major sequelae (neurological or renal) for life in a percentage that varies between 10 and 25% (4). Regardless of the final outcomes, the disease implies significant care-related burdens as in the acute phase more than 50% of patients require dialysis for a variable period of days or weeks, as well as often requiring resuscitative care (due to the neurological effects) and long or very long periods of hospitalization (5-9).

To date there are no specific treatments for hemolytic uremic syndrome from STEC and patient management relies on support measures only: red blood cell transfusions, antihypertensive therapy, dialysis, anticonvulsant drugs, respiratory and resuscitative care (10-13).

A strategy has recently been developed for managing fluids in patients affected by HUS that envisages the maximum expansion of the circulating volume to contrast the hypoxic/ischemic damage resulting from systemic thrombosis of the microcirculation. Such strategy has enabled fatality to be prevented and both acute complications of the disease, and long-term ones (neurological or renal) to be reduced, although they still remain high (14-19).

Considering the physiopathological basis of tissue damage in thrombotic microangiopathy, therefore also in HUS, it is plausible to consider that the vasodilation of the microcirculation can result in a further reduction in hypoxic/ischemic damage.

Fenoldopam, a dopamine receptor D₁ partial agonist, is known for its vasodilatory properties (20).

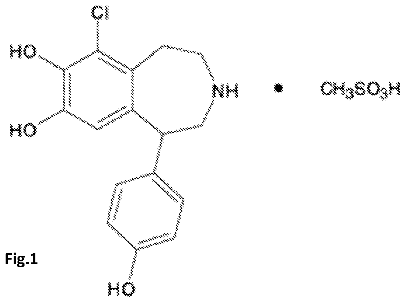

To date, it has usually been used in its salt form, Fenoldopam mesylate (see Figure 1), for treating hypertensive crises and, although off-label and at doses 10 times lower than the standard dosage, in acute renal failure (ARF), particularly in neonatal care with contrasting efficacy results, but without the observation of any side effects (21).

Fenoldopam mesylate is found on the market as an injectable preparation, containing the active ingredient Fenoldopam mesylate but also water, citric acid, propylene glycol, sodium citrate dihydrate, sodium metabisulfite.

Fenoldopam mesylate is a fast-acting vasodilator and bonds with weak affinity to α2 adrenergic receptors. It does not have any affinity for D2, alpha1 and 5HT1 and 5HT2 or muscarinic receptors and the vascular response is not uniform in the different vascular systems.

The author of the present invention has now found that Fenoldopam can be advantageously used in the treatment of severe forms of hemolytic uremic syndrome from STEC in paediatric patients treated from the diagnosis of the disease until normal platelet levels are reached with the immediate observation of a significant improvement that has brought about fast recovery for patients without any of the common complications mentioned above.

In particular, it has been observed that in light of its specific vasodilatory action in the context of thrombotic microangiopathy, Fenoldopam can be considered the drug of choice in the treatment of hemolytic uremic syndrome.

The present document highlights the results obtained in the 12 patients affected by hemolytic uremic syndrome and subjected to treatment with Fenoldopam within the context of the therapeutic support procedures.

Therefore, the subject matter of the present invention is Fenoldopam or its salts for use in the medical field in the treatment of hemolytic uremic syndrome (HUS), preferably in the secondary form due to Shiga-like toxin-producing Escherichia Coli (STEC-HUS) or in the atypical form (HUSa). Due to the analogy between the pathogenetic mechanisms of the kidney failure in the two pathologies, also the patient who will be later diagnosed for atypical HUS (HUSa), can be advantageously treated firstly with Fenoldopam according to the present invention. According to a preferred embodiment, the population of patients affected by hemolytic uremic syndrome (HUS) is the paediatric population.

According to a preferred embodiment of the invention the fenoldopam salt used is fenoldopam mesylate.

The administration of fenoldopam for use in the treatment of hemolytic uremic syndrome (HUS) takes place through continuous intravenous infusion.

Preferably, the treatment takes place through infusion administration of a dose of fenoldopam (preferably fenoldopam mesylate) comprised in the range 0.05-0.3 mcg/kg/min, preferably 0.1 mcg/kg/min.

Even more preferably the patients affected by HUS to be treated with fenoldopam (preferably fenoldopam mesylate) are patients with HUS and platelet levels < 150,000/mm³.

The subject matter of the present invention is also an injectable sterile aqueous solution comprising fenoldopam or its salts (preferably fenoldopam mesylate) together with one or more pharmaceutically acceptable excipients for use in the medical field in the treatment of hemolytic uremic syndrome, preferably from Shiga-like toxin-producing Escherichia Coli (STEC-HUS) or in the atypical form (HUSa).

According to a preferred embodiment of the present invention the injectable sterile aqueous solution for use in the treatment of hemolytic uremic syndrome is administered through the infusion of a dose of fenoldopam or its salts (preferably fenoldopam mesylate) comprised in the range 0.05-0.3 mcg/kg/min, preferably 0.1 mcg/kg/min.

The present invention will now be described, according to some preferred embodiments thereof, with particular reference to the attached figures, wherein:
- Figure 1 shows the chemical formula of fenoldopam mesylate;
- Figure 2 shows a graph of the trend of the median serum creatininemia, platelet count and need for dialysis (RRT) in patients affected by HUS subjected to standard treatment (fluid restriction), volume expansion or volume expansion + fenoldopam mesylate. Key - CI: confidence limit at the 95th percentile; EV: volume expansion; gg: days; pt: patient;
- Figure 3 shows the renal resistive index in 11 patients affected by HUS measured without Fenoldopam, during treatment with fenoldopam mesylate and after recovery.

In the following experimental section, the invention will be detailed according to a preferred embodiment.

### EXAMPLE 1: Clinical data obtained through treatment with fenoldopam mesylate of patients affected by HUS from Shiga-like toxin-producing Escherichia Coli (STEC)

### PATIENTS AND METHODS

The (14) patients affected by HUS from STEC cared for at the HUS Centre of the IRCCS "Ca' Granda Ospedale Maggiore Policlinico" Foundation in Milan were treated according to the invention with Fenoldopam mesylate starting from February 2017.

The Fenoldopam mesylate was administered in continuous infusion at a dosage comprised between 0.1-0.3 mcg/kg/min through peripheral vessel starting from the diagnosis of the disease.

For the purpose of the present analysis, hemolytic uremic syndrome from STEC was diagnosed by the concomitant presence of:
- Platelet consumption
- Non immune-mediated microangiopathic hemolysis
- Acute renal damage (kidney failure)
- Severe proteinuria (uProtein/uCreatinine ratio >2) or severe microscopic haematuria (>+++)
- Laboratory evidence of gastrointestinal infection associated with positive stool test for shiga toxin (Stx).

Cases of HUS associated with documented complement dysregulation and therefore treated as atypical HUS were excluded from the present analysis.

The patients included in the present study were also subjected to early circulating volume expansion according to the diagram previously described and published (14) to contrast hypoxic/ischemic damage.

In all the patients, treatment with Fenoldopam mesylate, and thus the infusion of isotonic fluids with the plasma, was always initiated within 3 hours of diagnosis of HUS from STEC (often before reaching our centre) and continued until improvement of the acute phase of the disease (platelet count >150,000/mm³).

The parents of all the patients were always informed as to the off-label nature of treatment with Fenoldopam and their informed consent for the treatment itself was acquired.

For the purpose of assessing the results obtained from the patient management procedure change, the following information was collected: personal data (age and gender), anthropometric data (weight), fundamental hematochemical data (hemoglobinemia, creatininemia, LDH and platelet count), clinical data (need for dialysis, neurological and/or other complications, need for ventilatory support, duration of hospitalization), hemodynamic data (renal resistive index both during treatment and after suspending treatment with Fenoldopam and again after recovery).

The data acquired was compared, retrospectively, with the natural course of the disease determined on 2 groups of patients already subject to previous analysis: Group 1. Patients treated with standard treatment (with fluid restriction)

Group 2. Patients treated with volume expansion only.

### STATISTICAL ANALYSIS

The data is expressed as mean and confidence limits to the 95th percentile (CI), median and interquartile range according to the variables and their Gaussian distribution.

ANOVA and Chi-square were used to compare continuous and categorical variables, respectively, in the three groups.

The analyses were performed using the software Stata 13 (StataCorp. 2013, Stata: Release 13. Statistical Software. Collage Station, TX: Stata Corp.LP).

### RESULTS

During 2017, a total of 14 cases of hemolytic uremic syndrome were treated with Fenoldopam mesylate according to the protocol detailed above.

Two of such patients were then affected by atypical HUS and therefore were excluded from the present analysis.

The trial treatment with Fenoldopam in association with volume expansion of patients with hemolytic uremic syndrome from STEC was shown to be extremely useful in the preservation of kidney function in 10 of the 12 subjects treated, even if all of them were in the most acute phase of the disease.

Only 2 subjects (17%) needed dialytic support during the acute phase of the disease (vs. 55 and 35% in patients respectively with volume restriction and those treated with volume expansion only). Moreover, the subjects that needed dialysis required it for a very contained number of days with respect to the past (1 and 4 days).

In a comparative analysis with respect to the 2 control groups, the mean days in dialysis which for the historic fluid restriction group was 4.9 (95th CI: 1.5-8.2) and in volume expansion only 2.4 (95th CI: 1.4-3.3), in the group treated with Fenoldopam it was 0.3 (CI: 0-1.1).

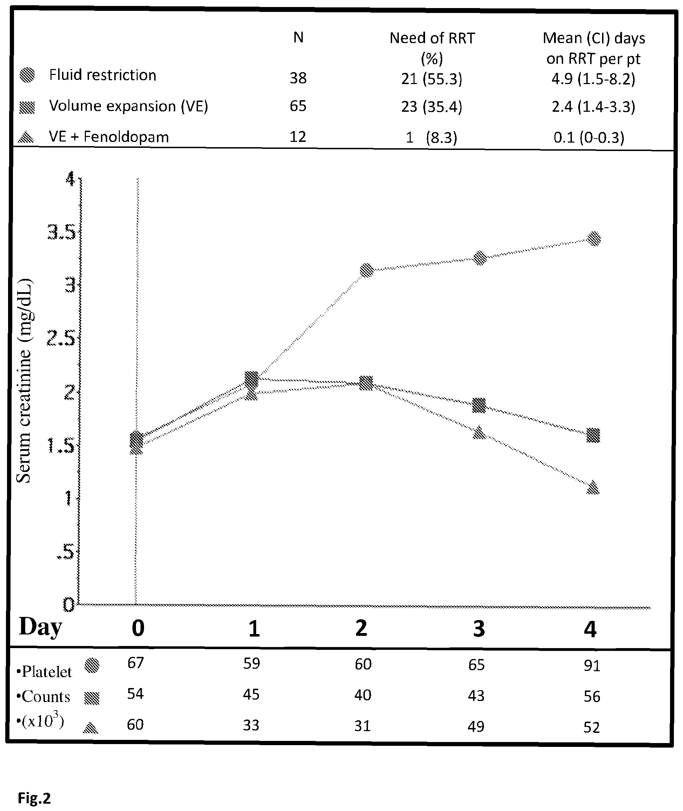

As highlighted in Figure 2, not only was the dialysis requirement lower in the group of patients treated with Fenoldopam, but the levels of creatininemia were also lower (compared with group 1) or equivalent (with group 2).

Moreover, all this took place during clearly active disease conditions as shown by the platelet levels during the first days of disease that were constantly and severely dropping, indicating still very severe disease (see Figure 2).

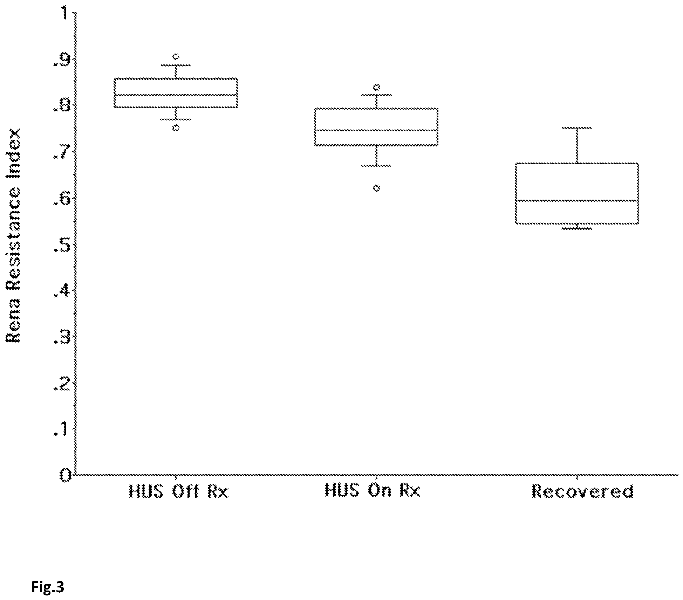

The analysis of the real resistive index (Figure 3) in "off' therapy and in "on" therapy with Fenoldopam (only available for the group treated) displayed a highly significant reduction both at statistical level (p<0.0001) and at biological level of the resistive index by Fenoldopam (-10.8%) hence indirectly demonstrating the increase in perfusion of the organ induced by treatment.

None of the children treated with Fenoldopam demonstrated any neurological effects during the acute phase, or permanent kidney or other damage. On this point, it is underlined that in the 2 compared groups, the nervous system involvement rate was 23.7% and 7.9% in relation to the restricted volume subjects and those exposed to expansion, while the permanent damage rate was 39.5% and 13.2% with 2 cases that led to patient death in the first group.

Finally, none of the children treated presented any side effects attributable to treatment with Fenoldopam mesylate.

In view of the initial difficulty of differential diagnosis between atypical HUS (HUSa) and HUS from STEC and the analogy of the pathogenetic mechanisms of the renal failure in the two pathologies, in later studies carried out in the years 2017-2018 also patients with atypical HUS were treated. Also in this case encouraging results were obtained, albeit the small group of treated patients.

More precisely, in three cases of HUSa (2 men and 1 woman) initially treated with Fenoldopam the natural course of the disease has been less aggressive of what generally observed, with the overall result that the patient reached the subsequent specific treatment with monoclonal antibody anti-C5, having accumulated less tissue damage, thus allowing a complete relief without permanent injuries. Therefore, also those patients who can later diagnosed for HUSa, can be advantageously be pretreated with Fenoldopam before starting with the specific therapy with anti-C5.

### REFERENCES

1. Loirat C, Frémeaux-Bacchi V. Orphanet Journal of Rare Disease. Vol. 6:60. 2011.
2. Griffin PM, Tauxe RV. Epidemiol. Rev. Vol.13:60-98, 1991.
3. Loos S, Ahlenstiel T, Kranz B, Staude H, Pape L, Härtel C, Vester U, Buchtala L, Benz K, Hoppe B, Beringer O, Krause M, Müller D, Pohl M, Lemke J, HillebrandG, Kreuzer M, König J, Wigger M, Konrad M, Haffner D, Oh J, Kemper MJ. Clin. Infect. Dis. Vol. 55(6):753-9, 2012.
4. Bale JF, Brasher C, Sigler RL. Am. J. Dis. Child Vol.134:869-872, 1980.
5. Garg AX, Suri RS, Barrowman N, Rehman F, Matsell D, Rosas-Arellano MP,Salvadori M, Haynes RB, Clark WF. JAMA. Vol. 290(10):1360-70, 2003.
6. Oakes RS, Siegler RL, McReynolds MA, Pysher T, Pavia AT. Vol. 117(5):1656-62. 2006.
7. Rooney JC, Anderson RM, Hopkins IJ (1971). Aust. Paediatr. J. Vol. 7:28-33.
8. Scheiring J, Andreoli SP, Zimmerhackl LB. Pediatr. Nephrol. Vol. 23:1749-1760. 2008.
9. Wong CS, Mooney JC, Brandt JR, Staples AO, Jelacic S, Boster DR, Watkins SL,Tarr PI. Clin. Infect. Dis. Vol. 55(1):33-41. 2012.
10. Iijima K, Kamioka I, Nozu K. Clin. Exp. Nephrol. Vol. 12(1):16-9, 2008.
11. Ikeda K, Ida O, Kimoto K, Takatorige T, Nakanishi N, Tatara K. Clin. Nephrol. Vol. 52(6):357-19, 1999.
12. Lapeyraque AL, Malina M, Fremeaux-Bacchi V, Boppel T, Kirschfink M, Oualha M, Proulx F, Clermont MJ, Le Deist F, Niaudet P, Schaefer F. N. Engl. J. Med. 2011 Jun 30; Vol. 364(26):2561-3. 2011.
13. Kielstein JT, Beutel G, Fleig S, Steinhoff J, Meyer TN, Hafer C, Kuhlmann U,Bramstedt J, Panzer U, Vischedyk M, Busch V, Ries W, Mitzner S, Mees S, Stracke S, Nürnberger J, Gerke P, Wiesner M, Sucke B, Abu-Tair M, Kribben A, Klause N, Schindler R, Merkel F, Schnatter S, Dorresteijn EM, Samuelsson O, Brunkhorst R; Nephrol. Dial. Transplant. Vol. 27(10):3807-15, 2012.
14. Ake JA, Jelacic S, Ciol MA, Watkins SL, Murray KF, Christie DL, Klein EJ, Tarr PI. Pediatrics. 115(6):e673-80, 2005.
15. Ardissino G, Tel F, Possenti I, Testa S, Consonni D, Paglialonga F et al. Pediatrics. 137(1): 1-9, 2016.
16. Balestracci A, Martin SM, Toledo I, Alvarado C, Wainsztein RE. Pediatr. Nephrol. Vol. 27(8):1407-10, 2012.
17. Grisaru S, Xie J, Samuel S, Hartling L, Tarr P, Schnadower D, Freedman S. JAMA Pediatrics. Vol. 171 (1):68-76, 2017.
18. Smith KE, Wilker PR, Reiter PL, Hedican EB, Bender JB, Hedberg CW. Pediatr. Infect. Dis. J. Vol.31(1):37-41. 2012.
19. Tarr PI, Gordon CA, Chandler WL. Lancet. Vol. 365(9464):1073-86. 2005.
20. Michael B, Murphy M, Clare Murray and George D. Shorten. N. Engl. J. Med.; Vol. 345:1548, 2001.
21. Gillies MA, Kakar V, Parker RJ, Honoré PM, Ostermann M. Crit. Care. Vol. 19:449, 2015

## Claims

1. Fenoldopam and/or its salts for use in medical field in the treatment of the hemolytic uremic syndrome.

2. Fenoldopam and/or its salts for use in medical field according to claim 1, wherein said salt is Fenoldopam mesylate.

3. Fenoldopam and/or its salts for use in medical field according to any one of claims 1-2, in the treatment of the hemolytic uremic syndrome caused by Shiga-like toxin-producing Escherichia Coli or of the atypical hemolytic uremic syndrome.

4. Fenoldopam and/or its salts for use in medical field according to any one of claims 1-3, in the treatment of the hemolytic uremic syndrome in the paediatric population.

5. Fenoldopam and/or its salts for use in medical field according to any one of the preceding claims, by intravenous injection or infusion administration.

6. Fenoldopam and/or its salts for use in medical field according to claim 5, by infusion administration of a dose of active principle comprised in the range 0.05-0.3 mcg/kg/min, preferably 0.1 mcg/kg/min.

7. Fenoldopam and/or its salts for use in medical field according to any one of the preceding claims, in patients having a platelet level <150.000 mm³.

8. Fenoldopam and/or its salts for use in medical field according to any one of the preceding claims, that is preceded by a treatment of early expansion of the circulating volume.

9. Injectable sterile aqueous solution comprising fenoldopam and/or its salts together with one or more pharmaceutically acceptable excipients for use in medical field in the treatment of the hemolytic uremic syndrome.

10. Injectable sterile aqueous solution for use according to claim 9, to be administered by infusion of a dose of active principle comprised in the range 0.05-0.3 mcg/kg/min, preferably 0.1 mcg/kg/min.

11. Injectable sterile aqueous solution for use according to any one of the claims 9-10, comprising Fenoldopam mesylate.

## Patentansprüche

1. Fenoldopam und/oder seine Salze zur Verwendung im medizinischen Bereich bei der Behandlung des hämolytisch-urämischen Syndroms.

2. Fenoldopam und/oder seine Salze zur Verwendung im medizinischen Bereich nach Anspruch 1, wobei das Salz Fenoldopam-Mesylat ist.

3. Fenoldopam und/oder seine Salze zur Verwendung im medizinischen Bereich nach einem der Ansprüche 1 bis 2 bei der Behandlung des hämolytisch-urämischen Syndroms, das durch Shiga-ähnliches Toxin produzierende Escherichia Coli verursacht wird, oder des atypischen hämolytisch-urämischen Syndroms.

4. Fenoldopam und/oder seine Salze zur Verwendung im medizinischen Bereich nach einem der Ansprüche 1 bis 3 bei der Behandlung des hämolytisch-urämischen Syndroms in der pädiatrischen Bevölkerung.

5. Fenoldopam und/oder seine Salze zur Verwendung im medizinischen Bereich nach einem der vorstehenden Ansprüche, verabreicht durch intravenöse Injektion oder Infusion.

6. Fenoldopam und/oder seine Salze zur Verwendung im medizinischen Bereich nach Anspruch 5 durch Infusionsverabreichung einer Dosis des Wirkstoffs im Bereich von 0,05 - 0,3 mcg/kg/min, vorzugsweise 0,1 mcg/kg/min.

7. Fenoldopam und/oder seine Salze zur Verwendung im medizinischen Bereich nach einem der vorstehenden Ansprüche bei Patienten mit einem Thrombozytenspiegel <150.000 mm³.

8. Fenoldopam und/oder seine Salze zur Verwendung im medizinischen Bereich nach einem der vorstehenden Ansprüche, dem eine Behandlung zur frühzeitigen Erweiterung des Kreislaufvolumens vorausgeht.

9. Injizierbare sterile wässrige Lösung, die Fenoldopam und/oder seine Salze zusammen mit einem oder mehreren pharmazeutisch akzeptablen Hilfsstoffen umfasst, zur Verwendung im medizinischen Bereich zur Behandlung des hämolytisch-urämischen Syndroms.

10. Injizierbare sterile wässrige Lösung zur Verwendung nach Anspruch 9, die durch Infusion einer Dosis des Wirkstoffs im Bereich von 0,05 - 0,3 mcg/kg/min, vorzugsweise 0,1 mcg/kg/min, zu verabreichen ist.

11. Injizierbare sterile wässrige Lösung zur Verwendung nach einem der Ansprüche 9 bis 10, umfassend Fenoldopam-Mesylat.

## Revendications

1. Fenoldopam et/ou ses sels pour une utilisation dans le domaine médical dans le traitement du syndrome hémolytique et urémique.

2. Fenoldopam et/ou ses sels pour une utilisation dans le domaine médical selon la revendication 1, dans lequel ledit sel est le mésylate de Fenoldopam.

3. Fenoldopam et/ou ses sels pour une utilisation dans le domaine médical selon l'une quelconque des revendications 1 à 2, dans le traitement du syndrome hémolytique et urémique provoqué par Escherichia Coli produisant une toxine de type Shiga ou du syndrome hémolytique et urémique atypique.

4. Fenoldopam et/ou ses sels pour une utilisation dans le domaine médical selon l'une quelconque des revendications 1 à 3, dans le traitement du syndrome hémolytique et urémique dans la population pédiatrique.

5. Fenoldopam et/ou ses sels pour une utilisation dans le domaine médical selon l'une quelconque des revendications précédentes, par administration par injection intraveineuse ou par perfusion.

6. Fenoldopam et/ou ses sels pour une utilisation dans le domaine médical selon la revendication 5, par administration par perfusion d'une dose de principe actif comprise dans la plage de 0,05 à 0,3 mcg/kg/min, de préférence de 0,1 mcg/kg/min.

7. Fenoldopam et/ou ses sels pour une utilisation dans le domaine médical selon l'une quelconque des revendications précédentes, chez des patients ayant un niveau de plaquettes < 150 000 mm³.

8. Fenoldopam et/ou ses sels pour une utilisation dans le domaine médical selon l'une quelconque des revendications précédentes, qui est précédée par un traitement d'expansion précoce du volume sanguin.

9. Solution aqueuse stérile injectable comprenant du fenoldopam et/ou ses sels conjointement avec un ou plusieurs excipients pharmaceutiquement acceptables pour une utilisation dans le domaine médical dans le traitement du syndrome hémolytique et urémique.

10. Solution aqueuse stérile injectable pour une utilisation selon la revendication 9, à administrer par perfusion d'une dose de principe actif comprise dans la plage de 0,05 à 0,3 mcg/kg/min, de préférence de 0,1 mcg/kg/min.

11. Solution aqueuse stérile injectable pour une utilisation selon l'une quelconque des revendications 9 à 10, comprenant du mésylate de Fenoldopam.
